# EUROPEAN PATENT APPLICATION

(11) **EP 4 332 986 A1**
(43) Date of publication of application: **06.03.2024**
(21) Application number: 22192521.7
(22) Date of filing: 29.08.2022
(51) Int. Cl.: G16H 50/30, G16H 50/20

(54) **METHOD AND SYSTEM FOR ASSESSING A PATIENT'S MEDICAL CONDITION**

(71) Applicant: BIOTRONIK SE & Co. KG, 12359 Berlin (DE)
(72) Inventor: Stoll, Alexandra, 13353 Berlin (DE); Dölitzsch, Christoph, 14055 Berlin (DE); Kallenberg, Felix, 10825 Berlin (DE)
(74) Representative: Biotronik Corporate Services SE

(57) **Abstract**

The invention relates to a computer-implemented method for assessing a patient's medical condition, comprising the steps of detecting first medical data (D1) of the patient by photoplethysmography (PPG), detecting second medical data (D2) of the patient by remote photoplethysmography (RPPG), detecting third medical data (D3) of the patient by vocal biomarker acquisition, and applying an algorithm (A) to the first medical data (D1), the second medical data (D2) and the third medical data (D3) for assessing the patient's medical condition, wherein the algorithm (A) outputs at least one numerical score (10) representing the patient's medical condition. The invention further relates to a system (1) for assessing a patient's medical condition.

## Description

The invention relates to a computer-implemented method for assessing a patient's medical condition.

Furthermore, the invention relates to a system for assessing a patient's medical condition.

Healthcare costs are rising and access to healthcare professionals is usually time-consuming and not readily available. When a symptom, concern or the need for a timely, quick and flexible health check arises, individuals currently mostly have to wait long and plan time accordingly in order to receive an appointment. In many cases of several medical conditions, the condition may only be diagnosed when the medical examination takes place at the moment when it occurs.

Few solutions currently exist, which enable individuals to assess their health without the presence of a healthcare professional. Current solutions are either focused on narrow application fields or do not provide adequate long-term vital sign tracking. Without the ability to provide users an overview of their vital sign development over time, these solutions do not account for sufficient comparability of individual results. Further, many of the solutions are likewise focused on the wellness market rather than setting the focus on achieving clinical accuracy.

US 9,042,971 B2 discloses a biometric monitoring device measuring various biometric information is provided that allows the person to take and/or display a heart rate reading by a simple user interaction with the device, e.g., by simply touching a heart rate sensor surface area or moving the device in a defined motion pattern. Some embodiments of this disclosure provide biometric monitoring devices that allow a person to get a quick heart rate reading without removing the device or interrupting their other activities. Some embodiments provide heart rate monitoring with other desirable features such as feedback on data acquisition status.

CN 107423549 B discloses a device configured to receive sensor data related to a user from a plurality of sensors. The device may include multiple types of sensors including an accelerometer, a heart rate sensor, a blood pressure sensor, a blood glucose sensor, a sweat sensor, one or more of a skin conductivity sensor or an imaging sensor, and a spectrometer. The device may process sensor data relating to the user from the multiple types of sensors to determine a health condition of the user. The device may provide information identifying a health condition of the user via a user interface based on processing sensor data relating to the user from the plurality of types of sensors.

It is therefore an object of the present invention to provide an improved method for assessing a patient's medical condition capable of providing a comprehensive assessment of an individual's cardiovascular health.

The object is solved by a computer implemented method for assessing a patient's medical condition having the features of claim 1. Furthermore, the object is solved by a system for assessing a patient's medical condition having the features of claim 13.

Moreover, the object is solved by a computer program with program code to perform the method according to the present invention having the features of claim 14. In addition, the object is solved by a computer-readable data carrier containing program code of a computer program for performing the method according to the present invention having the features of claim 15.

Further developments and advantageous embodiments are defined in the dependent claims.

The present invention provides a computer-implemented method for assessing a patient's medical condition. The method comprises detecting first medical data of the patient by photoplethysmography and detecting second medical data of the patient by remote photoplethysmography.

Furthermore, the method comprises detecting third medical data of the patient by vocal biomarker acquisition and applying an algorithm to the first medical data, the second medical data and the third medical data for assessing the patient's medical condition, wherein the algorithm outputs at least one numerical score representing the patient's medical condition. This algorithms on the basis of the input data from various methods of measurement may provide superior accuracy in assessing the patient's medical condition.

The present invention further provides a system for assessing a patient's medical condition. The system comprises a first sensor configured to detect first medical data of the patient by photoplethysmography and a second sensor configured to detect second medical data of the patient by remote photoplethysmography. The second sensor my be a camera or a camera sensor.

In addition, the system comprises a third sensor configured to detect third medical data of the patient by vocal biomarker acquisition and a computing device configured to apply an algorithm to the first medical data, the second medical data and the third medical data for assessing the patient's medical condition, wherein the algorithm is configured to output at least one numerical score representing the patient's medical condition.

The present invention moreover provides a computer program with program code to perform the method of the invention when the computer program is executed on a computer.

In addition, the present invention provides a computer-readable data carrier containing program code of a computer program for performing the method of the invention when the computer program is executed on a computer.

An idea of the present invention is to provide a method and system enabling a hassle-free assessment of an individual's health status at the time when needed. The method takes individual vital signs measurements from various technologies and combines these in an advanced score in order to provide users with a comprehensive assessment. Therewith the method increases the measurement's accuracy and improves the informative value for the user.

The method thus allows to assess an individual's cardiovascular health and identify the risk for cardiovascular and other diseases by measuring vital signs with smartphone-based technology.

Furthermore, the algorithm is applied to the vital signs measurements and thus determines cardiac arrhythmias, such as AFib or the worsening of heart failure. On the basis of these calculations, users can take early interventions in consultation with their healthcare professional and prevent the occurrence of consequential medical conditions, such as a stroke.

The algorithm may be embodied by a statistical algorithm or may comprise a neural network architecture trained by supervised learning methods.

The method of the present invention advantageously performed by an app of a smartphone and/or tablet computing device. The method combines standalone solutions and therefore provides a more accurate and more comprehensive health assessment for individuals.

Above all, the method aids at reducing overall healthcare costs through an early identification of diseases and contributes to an improvement of patient outcomes through earlier interventions.

According to an aspect of the invention, the photoplethysmography (PPG) detects a heart rate, a heart rate variability, a blood oxygen saturation, a blood pressure, and/or a cardiac output.

The following parameters may also be measured, detected or determined with the PPG: electrocardiogram (ECG), activity monitoring, sleep monitoring (light sleep, deep sleep, rapid eye movement sleep), heart rate variability (HRV), heart rate (with algorithms to detect atrial fibrillation), respiratory rate (asthma attack prediction, chronic obstructive pulmonary disease attack prediction), blood pressure (possible indicative for hypertension), oxygen saturation, systemic vascular resistance, cardiac stroke volume, cardiac output, cardiac index, body temperature, breathing volume, air flow, pulmonary fluid status, blood glucose, analyses to detect heart arrythmias (e.g. tachycardia and bradycardia), cardiac arrest, heart failure, sleep apnea, sepsis detection, chronic obstructive pulmonary disease (COPD).

In particular, with remote PPG, the following parameters may be measured, detected or determined: heart rate (possible indicative for cardiovascular, respiratory & general health), stress index (possible indicative for depression & anxiety), SD2 (PP descriptor quantifying long-term HRV), HRV (possible indicative for cardiovascular, diabetes, kidney, seizure diseases), oxygen saturation, parasympathetic activity, blood pressure (possible indicative for hypertension), pulse respiration quotient (may be used as triage and follow-up tool in cardiovascular disease recovery), respiratory rate, glucose level (possible indicative for diabetes), body weight, skin care & diseases, eyes common diseases, cough detection (possible indicative for lung cancer). Furthermore, analyses to derive heart attack risk, stroke risk, cardiovascular disease risk + hypercholesterolemia & Hypertriglyceridemia risk are possible by means of remote PPG.

PPG is an uncomplicated and inexpensive optical measurement method and is a non-invasive technology that uses a light source and a photodetector at the surface of skin to measure the volumetric variations of blood circulation.

Moreover, further valuable information can be extracted from the PPG signal in addition to heart rate estimation and pulse oximetry readings. PPG signal's second derivative wave contains important health-related information. Thus, analysis of this waveform can help clinicians to evaluate various cardiovascular-related diseases such as atherosclerosis and arterial stiffness. Moreover, investigating the second derivative wave of PPG signal can also assist in early detection and diagnosis of various cardiovascular illnesses that may possibly appear later in life. For early recognition and analysis of such illnesses, continuous and real-time monitoring is an important approach.

According to a further aspect of the invention, the remote photoplethysmography acquires the second medical data by analyzing the patient's vital parameters by means of a camera sensor, in particular of a smartphone and/or tablet computing device, facing the patient, said camera sensor detecting a blood pressure, a pulse, a respiratory rate and/or any parameter mentioned above. Remote photoplethysmography is thus a tool to measure heart rate remotely without attaching sensors. It only requires video recording with a high-resolution camera and can be beneficial in various kinds of physical, health and emotional monitoring.

According to a further aspect of the invention, the vocal biomarker acquisition detects a heart and/or lung condition (and/or a respiratory condition and/or metal condition of the patient, in particular a heart failure and/or a respiratory disease (such as COPD) and/or a mental disease. The vocal biomarker is thus understood to be a feature (or a combination of features) in the voice that has been identified and validated as associated with a clinical outcome.

According to a further aspect of the invention, the at least one numerical score outputted by the algorithm represents a cardiac arrythmia condition, an atrial fibrillation condition, a vascular assessment, a venous assessment, an arterial disease assessment, an arterial compliance and ageing assessment, a venous assessment, an endothelial function, a microvascular blood flow, a vasospastic condition, an autonomic function, a vasomotor function and thermoregulation and/or an orthostasis assessment. The algorithm thus integrates the first medical data, the second medical data and the third medical data into a score in order to provide users with a comprehensive assessment.

According to a further aspect of the invention, the algorithm further outputs numerical values representing an upper and lower threshold of a standard range for the patient's medical condition and/or historical data representing the patient's medical condition. The individual vital sign measurements are summarized in reports depicting the long-term development of the measurements. Together with educational information, this allows individuals to carry out health checks and track their health status compared to their individual baseline.

According to a further aspect of the invention, based on the outputted at least one numerical score representing the patient's medical condition (e.g. indication of deviation from the norm), an alert, a patient instruction and/or an instruction to a healthcare provider is generated if the at least one numerical score is outside the standard range for the patient's medical condition and/or deviates from the reacquired historical data by a predetermined amount. The method thus allows active sharing of data with healthcare professionals or in a private context.

According to a further aspect of the invention, the photoplethysmography is conducted by means of an infrared sensor of a smartphone and/or tablet computing device, and the vocal biomarker of the patient is acquired by a microphone of the smartphone and/or tablet computing device. All of the measurements taken can thus be performed by means of a smartphone and/or tablet computing device thus not requiring specialized medical equipment.

According to a further aspect of the invention, the infrared sensor of a smartphone and/or tablet computing device is configured to acquire the first medical data by means proximity to and or contact with a finger of the patient.

The PPG signal comprises pulsatile (AC) and superimposed (DC) components. The AC component is provided by the cardiac synchronous variations in blood volume that arise from heartbeats. The DC component is shaped by respiration, sympathetic nervous system activity, and thermoregulation. The AC component depicts changes in blood volume, which are caused by cardiac activity and depend on the systolic and diastolic phases. The systolic phase (also called, "rise time") starts with a valley and ends with the pulse wave systolic peak. The pulse wave end is marked by another valley at the end of the diastolic phase. Features such as rise time, amplitude, and shape can predict vascular changes in the bloodstream. Additionally, PPG can be used to measure HRV or the variations between heartbeat time intervals.

According to a further aspect of the invention, fourth medical data of the patient, in particular an EEG-signal is detected by an implantable medical device and/or a wearable device, said implantable medical device and/or wearable device being configured to acquire the fourth medical data of the patient continuously over at least 24 hours and/or in predetermined intervals. The method thus focuses on the supply of a perspicuous long-term vital sign reporting with adequate educational information. The method hence provides adequate long-term vital sign tracking.

According to a further aspect of the invention, the algorithm outputs a further numerical value representing a probability (and/or degree of severity) of the determined patient's medical condition. This provides the patient with additional information that allows for an improved assessment of the patient's medical condition.

According to a further aspect of the invention, the outputted data representing patient's medical condition is sent to a server of the healthcare provider automatically or through manual user selection. This allows for efficient further processing of the determined medical data representing the patient's medical condition.

According to some embodiments or a further aspect of the invention, which can be combined with other embodiments described herein, all above mentioned information and/or data may be stored in a database and/or a blockchain, and/or all above mentioned information and/or data may be secured using a blockchain and/or another suitable encryption scheme.

According to some embodiments, which can be combined with other embodiments described herein, the system and/or method further comprises and/or uses an artificial intelligence (AI) module integrated within the at least one processor and/or cooperating with the at least one processor, wherein the AI module is configured to process data stored in a database, blockchain or any other storage component. The AI module may be a software component and/or an algorithm comprising AI methods (e.g. machine learning models).

The herein described features of the computer-implemented method for assessing a patient's medical condition are also disclosed for the system for assessing a patient's medical condition, and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a flowchart of a computer-implemented method for assessing a patient's medical condition, according to the preferred embodiment of the invention; and
- Fig. 2: shows a diagram of a system for assessing a patient's medical condition according to a preferred embodiment of the invention.

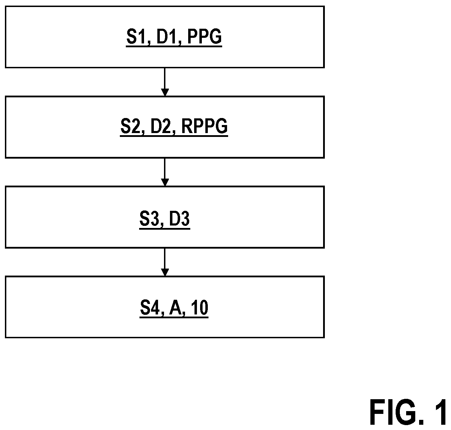

The computer-implemented method of Fig. 1 for assessing a patient's medical condition, comprises detecting first medical data D1 of the patient by photoplethysmography PPG and detecting second medical data D2 of the patient by remote photoplethysmography RPPG.

The method further comprises detecting third medical data D3 of the patient by vocal biomarker acquisition and applying an algorithm A to the first medical data D1, the second medical data D2 and the third medical data D3 for assessing the patient's medical condition, wherein the algorithm A outputs at least one numerical score 10 representing the patient's medical condition.

Additionally or alternatively, the method comprises detecting at least one of the first, second and third medica data D1, D2, D3.

The photoplethysmography PPG detects a heart rate, a heart rate variability, a blood oxygen saturation, a blood pressure, and/or a cardiac output.

The remote photoplethysmography RPPG acquires the second medical data D2 by analyzing the patient's vital parameters by means of a camera sensor, in particular of a smartphone 2 and/or tablet computing device 3, facing the patient, said camera sensor detecting a blood pressure, a pulse and/or a respiratory rate. The vocal biomarker acquisition further detects a heart and/or lung condition of the patient (which may be medical data D3), in particular a heart failure.

The at least one numerical score 10 outputted by the algorithm A represents a cardiac arrythmia condition, an atrial fibrillation condition, a vascular assessment, a venous assessment, an arterial disease assessment, an arterial compliance and ageing assessment, a venous assessment, an endothelial function, a microvascular blood flow, a vasospastic condition, an autonomic function, a vasomotor function and thermoregulation and/or an orthostasis assessment.

The algorithm A further outputs numerical values VI, V2, V3 representing an upper and lower threshold VI, V2 of a standard range for the patient's medical condition and/or historical data V3 representing the patient's medical condition.

Based on the outputted at least one numerical score 10 representing the patient's medical condition, an indication of a deviation from the norm, an alert, a patient instruction 12 and/or an instruction to a healthcare provider 14 is generated if the at least one numerical score 10 is outside the standard range for the patient's medical condition and/or deviates from the reacquired historical data V3 by a predetermined amount.

The photoplethysmography PPG is conducted by means of an infrared sensor 16 of a smartphone 2 and/or tablet computing device 3, and the vocal biomarker of the patient is acquired by a microphone 32 of the smartphone 2 and/or tablet computing device 3. The infrared sensor 16 of a smartphone 2 and/or tablet computing device 3 is further configured to acquire the first medical data D1 by means proximity to and or contact with a finger of the patient.

Fourth medical data D4 of the patient, in particular an EEG-signal is detected by an implantable medical device 22 and/or a wearable device 24, said implantable medical device 22 and/or wearable device 24 being configured to acquire the fourth medical data D4 of the patient continuously over at least 24 hours and/or in predetermined intervals.

The algorithm A outputs a further numerical value 26 representing a probability (and/or a degree) of the determined patient's medical condition (and/or severity). The outputted data representing patient's medical condition is further sent to a server 28 of the healthcare provider automatically or through manual user selection.

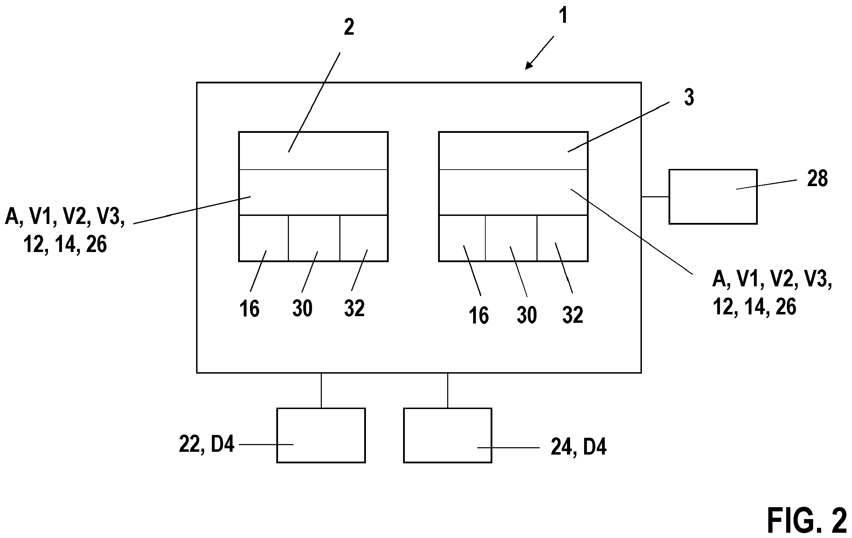

Fig. 2 shows a diagram of a system 1 for assessing a patient's medical condition according to a preferred embodiment of the invention.

The system 1 comprises a first sensor 16 configured to detect first medical data D1 of the patient by photoplethysmography PPG and a second sensor 30 configured to detect second medical data D2 of the patient by remote photoplethysmography RPPG. The second sensor 30 may be a camera or a camera sensor.

Moreover, the system 1 comprises a third sensor 32 configured to detect third medical data D3 of the patient by vocal biomarker acquisition and a computing device 34 configured to apply an algorithm A to the first medical data D1, the second medical data D2 and the third medical data D3 for assessing the patient's medical condition, wherein the algorithm A is configured to output at least one numerical score 10 representing the patient's medical condition.

Although specific embodiments have been illustrated and described herein, it will be understood by those skilled in the art that a variety of alternative and/or equivalent implementations exist. It should be noted that the exemplary embodiment or exemplary embodiments are examples only and are not intended to limit the scope, applicability, or configuration in any way.

Rather, the foregoing summary and detailed description provides those skilled in the art with convenient guidance for implementing at least one exemplary embodiment, it being understood that various changes in the functional scope and arrangement of the elements may be made without departing from the scope of the appended claims and their legal equivalents.

In general, this application intends to cover modifications or adaptations or variations of the embodiments disclosed herein. For example, an order of the method steps may be modified. The method may further be carried out sequentially or in parallel, at least in part.

### Reference Signs

- 1: system
- 2: smartphone
- 3: tablet computing device
- 10: numerical score
- 12: patient instruction
- 14: instruction to a healthcare provider
- 16: first sensor / infrared sensor
- 22: implantable medical device
- 24: wearable device
- 26: further numerical value
- 28: server
- 30: second sensor / camera sensor
- 32: third sensor / microphone
- A: algorithm
- D1: first medical data
- D2: second medical data
- D3: third medical data
- D4: fourth medical data
- PPG: photoplethysmography
- RPPG: remote photoplethysmography
- S1-S4: method steps
- V1, V2, V3: numerical values

## Claims

1. Computer-implemented method for assessing a patient's medical condition, comprising the steps of:
detecting (S1) first medical data (D1) of the patient by photoplethysmography (PPG); detecting (S2) second medical data (D2) of the patient by remote photoplethysmography (RPPG);
detecting (S3) third medical data (D3) of the patient by vocal biomarker acquisition; and
applying (S4) an algorithm (A) to the first medical data (D1), the second medical data (D2) and the third medical data (D3) for assessing the patient's medical condition, wherein the algorithm (A) outputs at least one numerical score (10) representing the patient's medical condition.

2. Computer-implemented method of claim 1, wherein the photoplethysmography (PPG) detects a heart rate, a heart rate variability, a blood oxygen saturation, a blood pressure, and/or a cardiac output.

3. Computer-implemented method of claim 1 or 2, wherein the remote photoplethysmography (RPPG) acquires the second medical data (D2) by analyzing the patient's vital parameters by means of a camera sensor (30), in particular of a smartphone (2) and/or tablet computing device (3), facing the patient, said camera sensor (30) detecting a blood pressure, a pulse and/or a respiratory rate.

4. Computer-implemented method of any one of the preceding claims, wherein the vocal biomarker acquisition detects a heart and/or lung condition of the patient, in particular a heart failure.

5. Computer-implemented method of any one of the preceding claims, wherein the at least one numerical score (10) outputted by the algorithm (A) represents a cardiac arrythmia condition, an atrial fibrillation condition, a vascular assessment, a venous assessment, an arterial disease assessment, an arterial compliance and ageing assessment, a venous assessment, an endothelial function, a microvascular blood flow, a vasospastic condition, an autonomic function, a vasomotor function and thermoregulation and/or an orthostasis assessment.

6. Computer-implemented method of any one of the preceding claims, wherein the algorithm (A) further outputs numerical values (V1, V2, V3) representing an upper and lower threshold (V1, V2) of a standard range for the patient's medical condition and/or historical data (V3) representing the patient's medical condition.

7. Computer-implemented method of claim 6, wherein based on the outputted at least one numerical score (10) representing the patient's medical condition, an indication of a deviation from the norm, an alert, a patient instruction (12) and/or an instruction to a healthcare provider (14) is generated if the at least one numerical score (10) is outside the standard range for the patient's medical condition and/or deviates from the reacquired historical data (V3) by a predetermined amount.

8. Computer-implemented method of any one of claims 3 to 7, wherein the photoplethysmography (PPG) is conducted by means of an infrared sensor (16) of a smartphone (2) and/or tablet computing device (3), and the vocal biomarker of the patient is acquired by a microphone (32) of the smartphone (2) and/or tablet computing device (3).

9. Computer-implemented method of any one of claim 8, wherein the infrared sensor (16) of a smartphone (2) and/or tablet computing device (3) is configured to acquire the first medical data (D1) by means proximity to and or contact with a finger of the patient.

10. Computer-implemented method of any one of the preceding claims, wherein fourth medical data (D4) of the patient, in particular an EEG-signal is detected by an implantable medical device (22) and/or a wearable device (24), said implantable medical device (22) and/or wearable device (24) being configured to acquire the fourth medical data (D4) of the patient continuously over at least 24 hours and/or in predetermined intervals.

11. Computer-implemented method of any one of the preceding claims, wherein the algorithm (A) outputs a further numerical value (26) representing a probability of the determined patient's medical condition.

12. Computer-implemented method of any one of the preceding claims, wherein the outputted data representing patient's medical condition is sent to a server (28) of the healthcare provider automatically or through manual user selection.

13. System (1) for assessing a patient's medical condition, comprising:
a first sensor (16) configured to detect first medical data (D1) of the patient by photoplethysmography (PPG);
a second sensor (30) configured to detect second medical data (D2) of the patient by remote photoplethysmography (RPPG);
a third sensor (32) configured to detect third medical data (D3) of the patient by vocal biomarker acquisition; and
a computing device (34) configured to apply an algorithm (A) to the first medical data (D1), the second medical data (D2) and the third medical data (D3) for assessing the patient's medical condition, wherein the algorithm (A) is configured to output at least one numerical score (10) representing the patient's medical condition.

14. Computer program with program code to perform the method of any one of claims 1 to 12 when the computer program is executed on a computer.

15. Computer-readable data carrier containing program code of a computer program for performing the method of to any one of claims 1 to 12 when the computer program is executed on a computer.
